# EUROPÄISCHE PATENTSCHRIFT

(11) **EP 1 675 851 B1**
(45) Veröffentlichungstag und Bekanntmachung des Hinweises auf die Patenterteilung: **20.08.2008**
(21) Anmeldenummer: 04765813.3
(22) Anmeldetag: 05.10.2004
(51) Int. Cl.: C07D 451/04

(54) **VERFAHREN ZUR HERSTELLUNG VON N-SUBSTITUIERTEN 3BETA-AMINONORTROPANEN**
METHOD FOR PRODUCING N-SUBSTITUTED 3BETA-AMINONORTROPANES
PROCEDE POUR PRODUIRE DES 3BETA-AMINONORTROPANES N-SUBSTITUES

(30) Priorität: 16.10.2003 DE 10348112; 18.03.2004 DE 102004013227
(43) Veröffentlichungstag der Anmeldung: 05.07.2006
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: SOBOTTA, Rainer, 55218 INGELHEIM (DE); IGNATOW, Hans-Peter, 55218 INGELHEIM (DE)
(74) Vertreter: Hammann, Heinz
(86) Internationale Anmeldenummer: PCT/EP2004/011094
(87) Internationale Veröffentlichungsnummer: WO 2005/037831

(56) Entgegenhaltungen:
- WO-A1-01/66521
- BURKS, JOHN E., JR. ET AL: "Development of a Manufacturing Process for Zatosetron Maleate" ORGANIC PROCESS RESEARCH & DEVELOPMENT , 1(3), 198-210 CODEN: OPRDFK; ISSN: 1083-6160, 1997, XP002314631
- PATENT ABSTRACTS OF JAPAN Bd. 013, Nr. 378 (E-809), 22. August 1989 (1989-08-22) -& JP 01 129571 A (NEC CORP), 22. Mai 1989 (1989-05-22)

## Beschreibung

### 1. TECHNISCHES GEBIET

Die Erfindung betrifft, ein Verfahren zur Herstellung von N-substituierten 3β-Aminonortropanen der Formel I 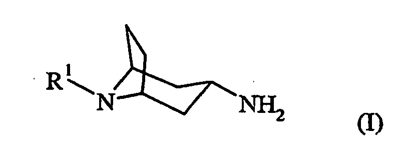 worin R¹ die in den Ansprüchen angegebene Bedeutung aufweist, aus dem entsprechenden 3-Oxonortropan bzw. dem entsprechenden 3 α-Aminonortropan, wobei man dieses mit einem Arylmethylamin bzw. einem Arylaldehyd in die entsprechenden Imine überführt, diese tautomerisiert bzw. isomerisiert und anschließend hydrolysiert.

### 2. STAND DER TECHNIK

Die Verbindungen der Formel I sind wertvolle Zwischenprodukte in der chemischen Synthese von verschiedenen pharmazeutischen Wirkstoffen (z.B. A. Nuhrich et al., Eur. J. Med. Chem. 31, 12, 1996, 957-964), bzw. stellen selbst pharmazeutische Wirkstoffe, insbesondere NMDA Rezeptor Modulatoren dar (z.B. A. H. Lewin et al., J. Med. Chem, 1998,41,988-995).

Durch Hydrierung von Tropanon-oximen in Gegenwart eines Platinkatalysators und eines Verdünnungsmittels unter erhöhtem Druck hydriert (z.B. S. Archer, J. Am. Chem. Soc. 80, 1958, 4677), werden N-substituierte 3-Aminonortropane überwiegend in der α-Form erhalten.

Durch Umsetzung von Tropanon-oximen mit metallischem Natrium in einem Alkohol (z.B. A. Nuhrich et al., *loc. cit)* entstehen überwiegend die β-Isomere der N-substituierte 3-Aminonortropane.

Allerdings eignet sich dieses Verfahren aus sicherheitstechnischen Gründen nur bedingt für eine großtechnische Produktion.

Aufgabe der vorliegenden Erfindung war es daher ein Herstellungsverfahren für N-substituierte 3β-Aminonortropane der Formel I aufzuzeigen, welche im großtechnischen Maßstab, in hohen Ausbeuten und günstigen Raum-Zeitausbeuten durchgeführt werden können. Weiterhin lag der Erfindung die Aufgabe zu Grunde, 3β-Aminonortropane der Formel I zur Verfügung zu stellen, die im Wesentlichen frei von den entsprechenden 3α-Isomeren sind.

Diese Aufgabe wurde erfindungsgemäß gelöst durch die Bereitstellung eines Verfahrens, wobei ausgehend von einem entsprechend substituierten 3-Oxonortropan oder einem entsprechend substituierten 3α-Aminonortropan das 3β-Aminonortropan erhalten wird.

Das erfindungsgemäße Verfahren erlaubt es, 3β-Aminonortropane im technischen Maßstab in hoher Ausbeute und Reinheit darzustellen.

### KURZE ZUSAMMENFASSUNG DER ERFINDUNG

Gegenstand der Erfindung ist ein Verfahren zur Herstellung von N-substituierten 3β-Aminonortropanen der Formel I oder eines Säureadditionssalzes davon, 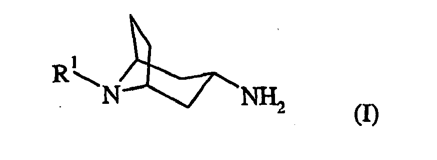 worin
R¹ für einen gegebenenfalls substituierten Rest ausgewählt aus der Gruppe bestehend aus C₁-C₈-Alkyl, C₂-C₈-Alkenyl, C₃-C₈-Cycloalkyl und C₆-C₁₀-Aryl-C₁-C₈-Alkyl steht,
wobei man entweder
(a) ein entsprechendes 3-Oxonortropan der Formel IIA 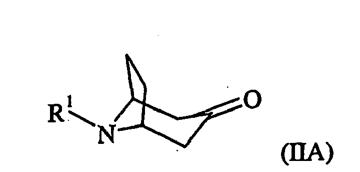 mit einem Arylmethylamin der Formel IIIA

   H₂N-CH₂-Ar (IIIA)

   umsetzt, worin Ar für einen gegebenenfalls substituierten Phenylrest oder einen gegebenenfalls substituierten 5- oder 6-gliedrigen Heteroaromatischen Rest mit mindestens einem Heteroatom ausgewählt aus der Gruppe N, O und S steht; oder
(b) ein entsprechendes 3α-Aminonortropan der Formel IIB 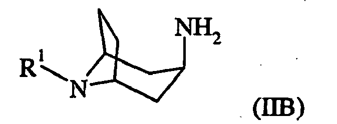 mit einem Arylaldehyd der Formel IIIB

   O=CH-Ar (IIIB)

   umsetzt;
das jeweils erhaltene Imin der Formeln IVA oder IVB 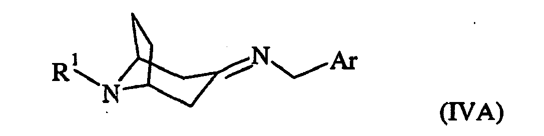 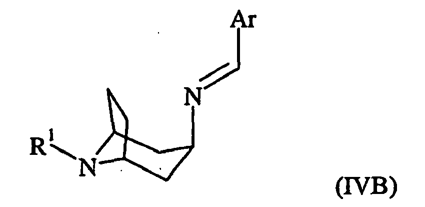 in Gegenwart einer Base in das thermodynamisch stabile Tautomer bzw. Isomer der Formel V 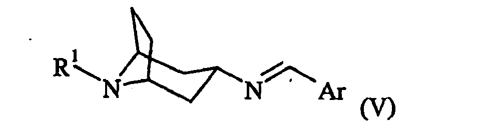 überführt; anschließend hydrolysiert und gegebenenfalls in ein Säureadditionssalz überführt.

Weiterhin betrifft die Erfindung die neuen Verbindungen der Formel V 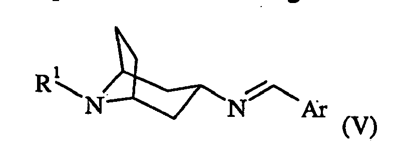 worin R¹ und Ar die angegebenen Bedeutungen aufweisen, sowie deren Tautomere bzw. Isomere der Formeln IVA und IVB.

### DETAILLIERTE BESCHREIBUNG DER ERFINDUNG

Der Begriff C₁-C₈-Alkyl allein oder in Zusammenhang mit anderen Gruppen oder Resten steht voranstehend und nachfolgend für eine geradkettige oder verzweigte Alkylgruppe mit 1 bis 8 C-Atomen, vorzugsweise 1 bis 6 C-Atomen, insbesondere 1 bis 4 C-Atomen. Beispielhaft seien genannt, Methyl, Ethyl, *n*-Propyl, Isopropyl, *n*-Butyl, *sec*-Butyl, Isobutyl, *tert*-Butyl, *n*-Pentyl, Neopentyl und *n*-Hexyl. Ganz besonders bevorzugt sind Methyl und Ethyl.

Der Begriff C₂-C₈-Alkenyl steht voranstehend und nachfolgend für eine geradkettige oder verzweigte Alkenylgruppe mit 2 bis 8 C-Atomen, vorzugsweise 2 bis 6 C-Atomen, insbesondere 2 bis 4 C-Atomen. Beispielhaft seien genannt, Vinyl, Prop-1-enyl, Prop-2-enyl (Allyl), But-1-enyl und *Pent-1-enyl.* Ganz besonders bevorzugt sind Vinyl und Allyl.

Der Begriff C₃-C₈-Cycloalkyl steht voranstehend und nachfolgend für eine Cycloalkylgruppe mit 3 bis 8 C-Atomen, vorzugsweise 3 bis 6 C-Atomen, insbesondere 5 oder 6 C-Atomen. Beispielhaft seien genannt, Cyclopropyl, Cyclobutyl, Cyclopentyl und Cyclohexyl.

Der Begriff C₆-C₁₀-Aryl allein oder in Zusammenhang mit anderen Gruppen oder Resten steht voranstehend und nachfolgend für eine gegebenenfalls durch C₁-C₆-Alkyl, C₁-C-₆-Alkoxy, Amino, C₁-C₆-Alkylamino, Di-(C₁-C₆-Alkyl)-amino oder Halogen substituierte Phenyl oder Naphthylgruppe, vorzugsweise für unsubstituiertes Phenyl oder für durch 1 bis 3 Substituenten ausgewählt aus der Gruppe bestehend aus C₁-C₄-Alkyl, C₁-C₄-Alkoxy, Amino, C₁-C₄-Alkylamino und Di-(C₁-C₄-Alkyl)-amino substituiertes Phenyl.

Die Gruppe Ar steht für eine wie voranstehend definierte Arylgruppe oder für einen unsubstituierten oder durch C₁-C₆-Alkyl, C₁-C₆-Alkoxy, Amino, C₁-C₆-Alkylamino, Di-(C₁-C₆-Alkyl)-amino oder Halogen substituierten 5- oder 6-gliedrigen heteroaromatischen Rest mit mindestens einem Heteroatom ausgewählt aus der Gruppe N, O, und S.

Vorzugsweise steht der heteroaromatische Rest für Pyrrol, Furan, Thiophen, Imidazol, Pyrazol, Thiazol, Thiadiazol, Tetrazol, Pyridin, Pyrimidin oder Pyrazin.

Bevorzugt ist das erfindungsgemäße Verfahren, wobei R¹ für einen Rest ausgewählt aus der Gruppe bestehend aus C₁-C₆ Alkyl, insbesondere Methyl oder Ethyl und Phenyl-C₁-C₃-Alkyl, insbesondere Benzyl steht.

Weiterhin bevorzugt ist das erfindungsgemäße Verfahren, wobei Ar für einen ein oder zweifach durch C₁-C₆-Alkoxy , insbesondere Methoxy, Ethoxy oder Isopropoxy und/oder Di-(C₁-C₆-Alkyl)-amino, insbesondere Dimethylamino oder Diethylamino substituierten Phenylrest steht. Ganz besonders bevorzugt befinden sich diese Substituenten in *para*Stellung in Bezug auf die Methylaminogruppe (Formel IIIA) oder die Carbaldehydgruppe (Formel IIIB).

Bevorzugt werden die einzelnen Schritte des erfindungsgemäße Verfahrens jeweils in einem inerten Verdünnungsmitteln ausgewählt aus der Gruppe bestehend aus gegebenenfalls halogenierten Kohlenwasserstoffen wie zum Beispiel Benzol, Toluol, Xylol, Methylcyclohexan oder Dichlormethan, Amiden wie zum Beispiel Acetamid oder Dimethylacetamid, Nitrilen wie zum Beispiel Acetonitril oder Propionitril, Sulfoxiden wie zum Beispiel Dimethylsulfoxid (DMSO), Ethern wie zum Beispiel Diethylether, *tert-*Butylmethylether (TBME) oder Tetrahydrofuran (THF) und Gemischen derselben durchgeführt.

In einer bevorzugten Ausführungsform des erfindungsgemäßen Verfahrens führt man die Reaktion zwischen der Verbindung der Formel IIA und der Verbindung der Formel IIIA bzw. zwischen der Verbindung der Formel IIB und der Verbindung der Formel IIIB unter dehydratisierenden Bedingungen durch.

In der Regel werden 0,5 bis 2,0 Äquivalente, vorzugsweise 0,8 bis 1,2 Äquivalente einer Verbindung der Formel IIA bzw. IIB mit bezogen auf ein Äquivalent der Formel IIIA bzw. IIIB umgesetzt. Vorzugsweise arbeitet man mit etwa äquimolaren Mengen an Verbindungen der Formel IIA bzw. IIB bezogen auf der Formel IIIA bzw. IIIB.

Als dehydratisierende Bedingungen seien genannt Umsetzungen in Gegenwart wasserbindender Agentien wie zum Beispiel Phosphorpentoxid, Titantetrachlorid oder einem Molekularsieb oder Umsetzung in Gegenwart einer anorganischen oder organischen Säure bei erhöhter Temperatur und Entfernung des gebildeten Wassers durch azeotrope Destillation. Besonders bevorzugt ist die Wasserabscheidung mit Hilfe von p-Toluolsulfonsäure (p-TsOH) in Toluol.

Die bei der Reaktion erhaltenen Imine der Formeln IVA oder IVB werden in Gegenwart einer Base in das Tautomer der Formel V überführt.

Besonders bevorzugt sind die neuen Imine der Formel V1 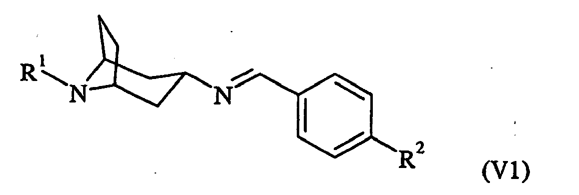 worin R¹ die für Formel I angegebene Bedeutung aufweist, und
R² für H, C₁-C₆-Alkoxy, insbesondere Methoxy oder Di-(C₁-C₆-Alkyl)-amino, insbesondere Dimethylamino steht.

Geeignete Basen sind starke Basen, insbesondere Metallalkoholate wie zum Beispiel Natrium Methanolat, Natrium Ethanolat, Kalium Ethanolat, Natrium *tert*-Butanolat, Kalium *tert*-Butanolat, Natrium Neopentanolat, Kalium Neopentanolat, insbesondere Kalium *tert*-Butanolat.

In der Regel wird die Isomerisierung in Gegenwart eines inerten Verdünnungsmittels durchgeführt. Bevorzugt sind polare, aprotische Lösungsmittel wie zum Beispiel Dimethylsulfoxid, Acetonitril, Dimethylacetamid, N,N-Dimethylethylenharnstoff (DEPU) N,N-Dimethylpropylenharnstoff (DMPU) oder Gemischen derselben. In einer besonders bevorzugten Ausführungsform wird die Reaktion mit Kalium *tert*-Butanolat in Gegenwart von THF und DMSO durchgeführt.

Die Reaktion wird in der Regel bei einer Temperatur von 0 bis 120 °C, vorzugsweise bei 25 bis 100 °C, insbesondere bei 40 bis 80 °C durchgeführt. Unter den angegebenen Bedingungen ist die Isomerisierung in 1 bis 15 Stunden beendet.

In der Regel führt man die Hydrolyse der Verbindung der Formel IV in Gegenwart einer Säure durch. Bevorzugt sind starke anorganische oder organische Säuren, wie zum Beispiel Schwefelsäure, Salzsäure, Phosphorsäure oder Trifluoressigsäure. Besonders bevorzugt führt man die Hydrolyse in einem Zweiphasensystem bestehend aus einem organischen, mit Wasser wenig mischbaren Lösungsmittel, insbesondere Toluol oder Dichlormethan, Wasser und der entsprechenden Säure, insbesondere Schwefelsäure durch.

Die Hydrolyse wird in der Regel bei einer Temperatur von 0 bis 100 °C, vorzugsweise bei 10 bis 60 °C, insbesondere bei 15 bis 30 °C durchgeführt. Unter den angegebenen Bedingungen ist die Hydrolyse in 15 bis 360 Minuten beendet.

In einer besonders bevorzugten Ausführungsform wird die wässrige Phase des so erhaltenen Rohprodukts abgetrennt, und mit einer geeigneten Base, vorzugsweise einem Alkalihydroxid, insbesondere mit Natronlauge schwach alkalisch gestellt, vorzugsweise auf einen pH-Wert von 7,5 bis 9,5, insbesondere etwa 8,0. Die so erhaltene wässrige Phase wird mit einem mit Wasser nicht mischbarem Lösungsmittel, vorzugsweise einem aliphatischen oder aromatischen Kohlenwasserstoff, insbesondere Toluol extrahiert. Die so erhaltene organische Phase kann zur Rückgewinnung von Ausgangsmaterialien verwendet werden.

Die wässrige Phase wird abgetrennt, und mit einer geeigneten Base, vorzugsweise einem Alkalihydroxid, insbesondere mit Natronlauge stark alkalisch gestellt, vorzugsweise auf einen pH-Wert von 10,0 bis 14,0, insbesondere etwa 12,7. Die so erhaltene wässrige Phase wird mit einem mit Wasser nicht mischbarem Lösungsmittel, vorzugsweise einem gegebenenfalls halogenierten, aliphatischen oder aromatischen Kohlenwasserstoff, insbesondere Toluol oder Dichlormethan extrahiert. Die so erhaltenen vereinten organischen Phasen werden konzentriert. Man erhält so die Verbindung der Formel I in Form der freien Base.

Diese kann nach an sich bekannten Verfahren durch Behandeln mit einer anorganischen oder organischen Säure in das entsprechende Säureadditionssalz überführt werden. Vorzugsweise wird die freie Base dazu in einem polaren Lösungsmittel, vorzugsweise einem Alkohol wie zum Beispiel Methanol, Ethanol oder Isopropanol, Wasser oder einem Gemisch davon, insbesondere einem Gemisch aus Ethanol und Wasser aufgenommen und mit der entsprechenden Säure, vorzugsweise einer anorganischen Säure wie Salzsäure oder Schwefelsäure auf einen schwach basischen pH Wert eingestellt.

Der sich dabei bildende Niederschlag des Salzes wird abgetrennt, mit einem polaren Lösungsmittel gewaschen und getrocknet. Man erhält so das Produkt in reiner kristalliner Form ohne weitere Aufreinigung.

Weitere vorteilhafte Aspekte der erfindungsgemäßen Vorgehensweise sind die hohe Raum-Zeit-Ausbeute beim vorliegenden Prozess sowie die hohe Ausbeute und Reinheit an den jeweiligen Zwischenprodukten, welche ohne chromatographische Aufreinigung weiterverarbeitet werden können.

Die nachfolgenden Beispiele dienen der Illustration exemplarisch durchgeführter Verfahren zur Herstellung der Verbindung der Formel I. Sie sind lediglich als mögliche, exemplarisch dargestellte Vorgehensweisen zu verstehen, ohne die Erfindung auf deren Inhalt zu beschränken.

### Beispiel 1

### N-Methyl-3-β-aminonortropan Hemisulfat

Ein Gemisch aus 17,0 g N-Methyl-3-α-aminonortropan, 18,7 g 4-Dimethylaminobenzaldehyd, 125 ml Toluol und 0,125g p-TsOH wird am Wasserabscheider 5 Stunden zum Sieden erhitzt. Das Rektionsgemisch wird eingeengt. Man erhält 36,2 g N-Methyl-3-α-(4-dimethylaminobenzylidenamino)-tropan, welches ohne weitere Aufreinigung weiterverarbeitet wird.

Ein Gemisch von 36,2 g N-Methyl-3-α-(4-dimethylaminobenzylidenamino)-tropan, 100 ml DMSO, 13,8 g einer 24 %-igen Lösung von Kalium tert-Butanolat in THF wird 10 Stunden auf 65 bis 70°C erhitzt. Das Reaktionsgemisch wird in 500 ml Acetonitril eingerührt und filtriert mit Acetonitril gewaschen. Der Filterrückstand wird in 300 ml Toluol gelöst und langsam in ein Gemisch aus 200 ml Wasser und 10 ml konzentrierte Schwefelsäure eingerührt.

Nach 45 minütigem Rühren bei Raumtemperatur werden die Phasen getrennt, die wässrige Phase wird mit Natronlauge auf pH 8,0 gestellt und mit 100 ml Toluol gewaschen. Die Phasen werden getrennt und die wässrige Phase wird mit 150 ml Dichlormethan versetzt und mit Natronlauge auf pH 12,9 gestellt, die Phasen werden getrennt und die wässrige Phase wird dreimal mit 150 ml Dichlormethan extrahiert. Die vereinten organischen Phasen werden konzentriert. Man erhält 8,8 g eines braunen Öls. Dieses Öl wird in 150 ml Ethanol aufgenommen, mit halbkonzentrierter Schwefelsäure auf pH 7,5-6,5 gestellt und 1 Stunde bei Raumtemperatur gerührt. Man erhält das 14,45 g des Salzes mit einem Zersetzungspunkt von 320 °C.

### Beispiel 2

### N-Methyl-3-β-aminonortropan Hemisulfat

Ein Gemisch aus 17,0 g N-Methyl-3-α-aminonortropan, 17,0 g 4-Methoxybenzaldehyd, 125 ml Toluol und 0,125g p-TsOH wird am Wasserabscheider 5 Stunden zum Sieden erhitzt. Das Rektionsgemisch wird eingeengt. Man erhält 33,5 g N-Methyl-3-α-(4-methoxybenzylidenamino)-tropan, welches ohne weitere Aufreinigung weiterverarbeitet wird.

Ein Gemisch von 33,5 g N-Methyl-3-α-(4-methoxybenzylidenamino)-tropan, 100 ml DMSO, 8,5 g einer 24 %-igen Lösung von Kalium *tert*-Butanolat in THF wird 5 Stunden auf 65 bis 70 °C erhitzt. Das Reaktionsgemisch wird in ein Gemisch aus 500 ml Acetonitril und 600 ml Wasser eingerührt, mit dreimal 300 ml Toluol extrahiert und konzentriert. Der Rückstand wird in 200 ml Wasser aufgenommen, die wässrige Phase wird mit dreimal 200 ml Dichlormethan extrahiert. Die vereinten Extrakte werden konzentriert, in 200 ml Toluol aufgenommen und in eine Lösung von 10 ml konzentrierter Schwefelsäure in 300 ml Wasser getropft.

Nach 45 minütigem Rühren bei Raumtemperatur werden die Phasen getrennt, die wässrige Phase wird mit Natronlauge auf pH 8,0 gestellt, mit 50 ml Toluol und zweimal 50 ml Dichlormethan extrahiert. Die wässrige Phase wird mit 150 ml Dichlormethan versetzt und mit Natronlauge auf pH 12,9 gestellt. Die Phasen werden getrennt und die wässrige Phase wird mit 6 mal 60 ml Dichlormethan extrahiert. Die vereinten organischen Phasen werden über Natriumsulfat getrocknet, filtriert und konzentriert. Man erhält 17,1 g eines goldbraunen Öls. Dieses Öl wird in 200 ml Ethanol aufgenommen, mit halbkonzentrierter Schwefelsäure auf pH 7,0-6,5 gestellt und 30 Minuten bei Raumtemperatur gerührt. Man erhält das 17,5 g des Salzes mit einem Zersetzungspunkt von 320 °C.

### Beispiel 3

### N-Benzyl-3-β-aminonortropan Hemisulfat

Ein Gemisch aus 75,5 g N-Benzyl-nortropinon-hydrochlorid, 60 ml Wasser, 173 ml Toluol und 22,5 ml Natronlauge wird bei Raumtemperatur gerührt. Die Phasen werden getrennt und die wässrige Phase wird mit 87 ml Toluol extrahiert. Ein Gemisch der vereinten Toluolphasen, 41,2 g 4-Methoxybenzylamin und 0,22 g p-TsOH wird am Wasserabscheider 5 Stunden zum Sieden erhitzt. Das Rektionsgemisch wird eingeengt. Man erhält 95 g N-Benzyl-3-(4-mehoxybenzylimino)-tropan, welches ohne weitere Aufreinigung weiterverarbeitet wird.

Ein Gemisch von 95 g N-Benzyl-3-(4-mehoxybenzylimino)-tropan, 240 ml DMSO, 16,8 g einer 24 %-igen Lösung von Kalium tert-Butanolat in THF wird 5 Stunden auf 65 bis 70 °C erhitzt. Das Reaktionsgemisch wird zwischen 300 ml Toluol und 900 ml Wasser verteilt, die wässrige Phase wird zweimal mit jeweils 300 ml Toluol extrahiert. Die vereinten Toluolphasen werden langsam in ein Gemisch aus 960 ml Wasser und 21,7 ml konzentrierte Schwefelsäure eingerührt.

Nach 60 minütigem Rühren bei Raumtemperatur werden die Phasen getrennt, die wässrige Phase wird die wässrige Phase wird mit Natronlauge auf pH 8,0 gestellt, mit 300 ml Toluol gewaschen und mit Kochsalz gesättigt. Die wässrige Phase wird mit 150 ml Dichlormethan versetzt und mit Natronlauge auf pH 12,9 gestellt, die Phasen werden getrennt und die wässrige Phase wird dreimal mit 150 ml Toluol extrahiert. Die vereinten organischen Phasen werden konzentriert. Man erhält 54,5 g des Produkts in Form eines braunen Öls.

Dieses wird in einem Gemisch aus 330 ml Ethanol und 6,67 ml Wasser aufgenommen und mit 35 ml einer 32 %-igen Schwefelsäure auf pH 8,6 eingestellt. Der gebildete Niederschlag wird filtriert und mit 265 ml Ethanol gewaschen. Der erhaltene Feststoff wird bei 50 °C getrocknet. Man erhält 53,8 g des Salzes in Form weißer Kristalle mit einem Schmelzpunkt von 283,4-284,9 °C.

### Beispiel 4

### N-Benzyl-3-β-aminonortropan Hemisulfat

Ein Gemisch aus 89,5 g N-Benzyl-3-α-aminonortropan dihydrochlorid-hemihydrat, 100 ml Wasser und 172 ml Toluol wird mit 45 ml einer 45 %-igen Natronlauge versetzt und gerührt. Die Phasen werden separiert und die wässrige Phase mit 86 ml Toluol extrahiert. Die vereinten Toluolphasen werden mit 44,8 g 4-Dimethylaminobenzaldehyd und 0,22 g p-TsOH versetzt und am Wasserabscheider 4 Stunden zum Sieden erhitzt. Das Rektionsgemisch wird eingeengt: Man erhält 104,2 g N-Benzyl-3-α-(4-dimethylaminobenzylidenamino)-tropan, welches ohne weitere Aufreinigung weiterverarbeitet wird.

Ein Gemisch von 104,2 g N-Benzyl-3-α-(4-dimethylaminobenzylidenamino)-tropan, 239 ml DMSO, 24 g einer 24 %-igen Lösung von Kalium *tert*-Butanolat in THF wird 4 Stunden und 15 Minuten auf 65 bis 70 °C erhitzt. Das Reaktionsgemisch wird zwischen 300 ml Toluol und 900 ml Wasser, in dem 21 g Kochsalz gelöst sind, verteilt, die wässrige Phase wird zweimal mit jeweils 300 ml Toluol extrahiert. Die vereinten Toluolphasen werden mit 300 ml Wasser, in dem 7 g Kochsalz gelöst sind, gewaschen. Die so erhaltene organische Phase wird langsam in ein Gemisch aus 875 ml Wasser und 21,8 ml konzentrierte Schwefelsäure eingerührt.

Nach 60 minütigem Rühren bei Raumtemperatur werden die Phasen getrennt, die wässrige Phase wird mit Natronlauge auf pH 8,0 gestellt und mit 300 ml Toluol gewaschen. Die Phasen werden getrennt und die wässrige Phase wird mit 300 ml Toluol versetzt und mit Natronlauge auf pH 12,7 gestellt. Die organische Phase wird abgetrennt, und die wässrige Phase wird zweimal mit 300 ml Toluol extrahiert. Die vereinten organischen Phasen der Extraktion bei pH 12,7 werden konzentriert. Man erhält 58,77 g (90,6 % d.Th.) des Produkts in Form eines gelblichen Öls.

Dieses wird in einem Gemisch aus 330 ml Ethanol und 6,67 ml Wasser aufgenommen und mit 35 ml einer 32 %-igen Schwefelsäure auf pH 8,6 eingestellt. Der gebildete Niederschlag wird filtriert und mit 265 ml Ethanol gewaschen. Der erhaltene Feststoff wird bei 50 °C getrocknet. Man erhält 66,65 g (83,7 % d. Th.) des Salzes in Form weißer Kristalle mit einem Schmelzpunkt von 283,4-284,9 °C.

## Patentansprüche

1. Verfahren zur Herstellung von N-substituierten 3β-Aminonortropanen der Formel I oder eines Säureadditionssalzes davon, 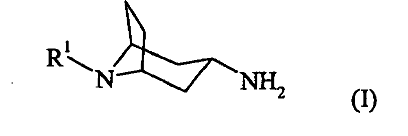 worin
R¹ für einen gegebenenfalls substituierten Rest ausgewählt aus der Gruppe bestehend aus C₁-C₈-Alkyl, C₂-C₈-Alkenyl, C₃-C₈-Cycloalkyl und C₆-C₁₀-Aryl-C₁-C₈-Alkyl steht,
**dadurch gekennzeichnet, dass** man entweder
(a) ein entsprechendes 3-Oxonortropan der Formel IIA 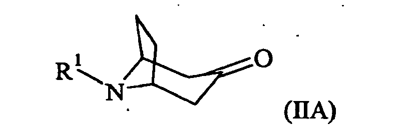 mit einem Arylmethylamin der Formel IIIA
H₂N-CH₂-Ar (IIIA)
umsetzt, worin
Ar für einen gegebenenfalls substituierten Phenylrest oder einen gegebenenfalls substituierten 5- oder 6-gliedrigen Heteroaromatischen Rest mit mindestens einem Heteroatom ausgewählt aus der Gruppe N, O und S steht; oder
(b) ein entsprechendes 3α-Aminonortropan der Formel IIB 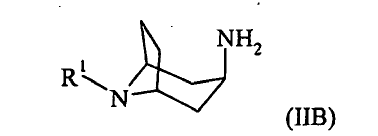 mit einem Arylaldehyd der Formel IIIB
O=CH-Ar (IIIB)
umsetzt;
das jeweils erhaltene Imin der Formeln IVA oder IVB 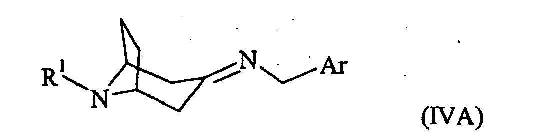 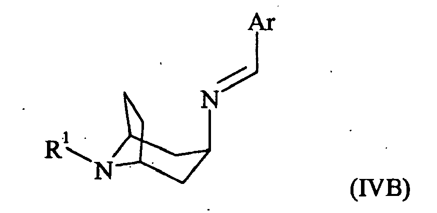 in Gegenwart einer Base in das Tautomer bzw. Isomer der Formel V überführt; 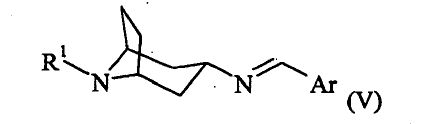 anschließend hydrolysiert und gegebenenfalls in ein Säureadditionssalz überführt.

2. Verfahren nach Anspruch 1, **dadurch gekennzeichnet, dass** R¹ für einen Rest ausgewählt aus der Gruppe bestehend aus C₁-C₆-Alkyl und Phenyl-C₁-C₃-Alkyl steht.

3. Verfahren nach Anspruch 1 oder 2, **dadurch gekennzeichnet, dass** Ar für einen ein oder zweifach durch C₁-C₆-Alkoxy und/oder Di-(C₁-C₆-Alkyl)-amino substituierten Phenylrest steht.

4. Verfahren nach einem der Ansprüche 1 bis 3, **dadurch gekennzeichnet, dass** man die Reaktion zwischen der Verbindung der Formel IIA und der Verbindung der Formel IIIA bzw. zwischen der Verbindung der Formel IIB und der Verbindung der Formel IIIB unter dehydratisierenden Bedingungen durchführt.

5. Verfahren nach einem der Ansprüche 1 bis 4, **dadurch gekennzeichnet, dass** man die Hydrolyse der Verbindung der Formel V in Gegenwart einer Säure durchführt.

6. Verfahren nach einem der Ansprüche 1 bis 5, **dadurch gekennzeichnet, dass** die einzelnen Schritte jeweils in einem inerten Verdünnungsmitteln ausgewählt aus der Gruppe bestehend aus gegebenenfalls halogenierten Kohlenwasserstoffen, Amiden, Nitrilen, Sulfoxiden, Ethern und Gemischen derselben durchgeführt werden.

7. Ein N-substituiertes 3-β-(Arylmethyliden)-aminonortropan der Formel V 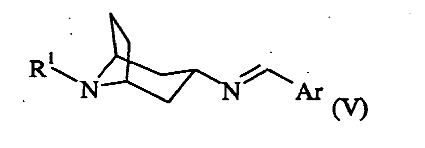 worin
R¹ für einen gegebenenfalls substituierten Rest ausgewählt aus der Gruppe bestehend aus C₁-C₈-Alkyl, C₂-C₈-Alkenyl, C₃-C₈-Cycloalkyl und C₆-C₁₀-Aryl-C₁-C₈-Alkyl steht; und
Ar für einen gegebenenfalls substituierten Phenylrest oder einen gegebenenfalls substituierten 5- oder 6-gliedrigen Heteroaromatischen Rest mit mindestens einem Heteroatom ausgewählt aus der Gruppe N, O und S steht.

8. Ein 3-β-(Arylmethyliden)-aminonortropan der Formel V nach Anspruch 7, worin
R¹ für einen Rest ausgewählt aus der Gruppe bestehend aus C₁-C₆-Alkyl und Phenyl-C₁-C₃-Alkyl steht, und
Ar für unsubstituiertes Phenyl oder einen ein oder zweifach durch C₁-C₆-Alkoxy und/oder Di-(C₁-C₆-Alkyl)-amino substituierten Phenylrest steht.

## Claims

1. Process for preparing N-substituted 3β-aminonortropanes of formula I or an acid addition salt thereof, 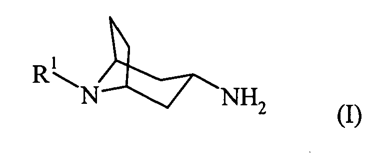 wherein
R¹ denotes an optionally substituted group selected from the group consisting of C₁-C₈-alkyl, C₂-C₈-alkenyl, C₃-C₈-cycloalkyl and C₆-C₁₀-aryl-C₁-C₈-alkyl,
**characterised in that** either
(a) a corresponding 3-oxonortropane of formula IIA 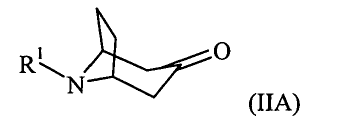 is reacted with an arylmethylamine of formula IIIA
H₂N-CH₂-Ar (IIIA)
wherein
Ar denotes an optionally substituted phenyl group or an optionally substituted 5- or 6-membered heteroaromatic group with at least one heteroatom selected from the group N, O and S; or
(b) a corresponding 3α-aminonortropane of formula IIB 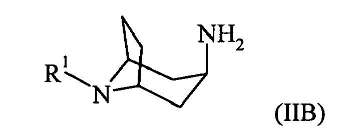 is reacted with an arylaldehyde of formula IIIB
O=CH-Ar (IIIB);
the imine of formulae IVA or IVB 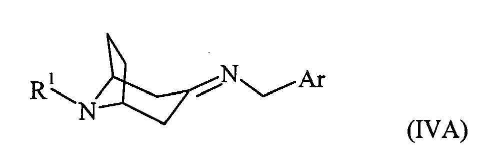 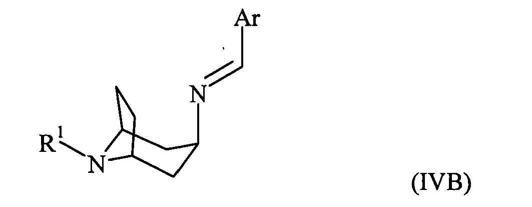 obtained in each case is converted, in the presence of a base, into the tautomer or isomer of formula V 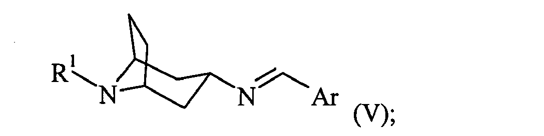 then hydrolysed and optionally converted into an acid addition salt.

2. Process according to claim 1, **characterised in that** R¹ denotes a group selected from the group consisting of C₁-C₆-alkyl and phenyl-C₁-C₃-alkyl.

3. Process according to claim 1 or 2, **characterised in that** Ar denotes a phenyl group mono- or disubstituted by C₁-C₆-alkoxy and/or di-(C₁-C₆-alkyl)-amino.

4. Process according to one of claims 1 to 3, **characterised in that** the reaction between the compound of formula IIA and the compound of formula IIIA or between the compound of formula IIB and the compound of formula IIIB is carried out under dehydrating conditions.

5. Process according to one of claims 1 to 4, **characterised in that** the hydrolysis of the compound of formula V is carried out in the presence of an acid.

6. Process according to one of claims 1 to 5, **characterised in that** the individual steps in each case are carried out in an inert diluent selected from the group consisting of optionally halogenated hydrocarbons, amides, nitriles, sulphoxides, ethers and mixtures thereof.

7. An N-substituted 3-β-(arylmethylidene)-aminonortropane of formula V 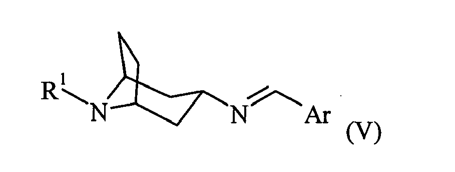 wherein
R¹ denotes an optionally substituted group selected from the group consisting of C₁-C₈-alkyl, C₂-C₈-alkenyl, C₃-C₈-cycloalkyl and C₆-C₁₀-aryl-C₁-C₈-alkyl; and
Ar denotes an optionally substituted phenyl group or an optionally substituted 5- or 6-membered heteroaromatic group with at least one heteroatom selected from the group N, O and S.

8. A 3-β-(arylmethylidene)-aminonortropane of formula V according to claim 7, wherein
R¹ denotes a group selected from the group consisting of C₁-C₆-alkyl and phenyl-C₁-C₃-alkyl, and
Ar denotes unsubstituted phenyl or a phenyl group mono- or disubstituted by C₁-C₆-alkoxy and/or di-(C₁-C₆-alkyl)-amino.

## Revendications

1. Procédé pour préparer des 3β-aminonortropanes N-substitués de formule 1 ou un sel d'addition d'acide de ceux-ci, 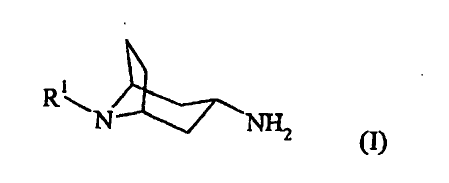 où
R¹ représente un groupement éventuellement substitué choisi dans le groupe consistant en C₁-C₈-alkyle, C₂-C₈-alcényle, C₃-C₈-cycloalkyle et C₆-C₁₀-aryl-C₁-C₈-alkyle,
**caractérisé en ce que**
(a) on fait réagir un 3-oxonortropane correspondant de formule IIA 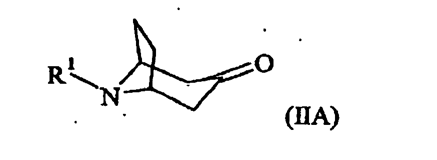 avec une arylméthylamine de formule IIIA
H₂N-CH₂-Ar (IIIA)
où
Ar représente un groupement phényle éventuellement substitué ou un groupement hétéroaromatique à 5 ou 6 chaînons éventuellement substitué ayant au moins un hétéroatome choisi dans le groupe N, O et S ; ou
(b) on fait réagir un 3α-aminonortropane correspondant de formule IIB 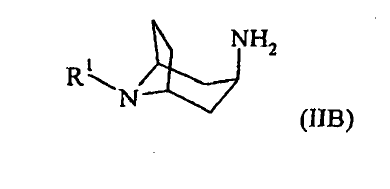 avec un arylaldéhyde de formule IIIB
O=CH-Ar (IIIB) ;
on convertit l'imine de formule IVA ou IVB obtenue 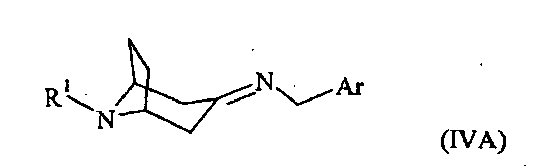 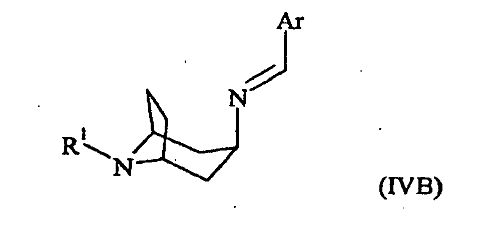 en présence d'une base en le tautomère ou l'isomère de formule V ; 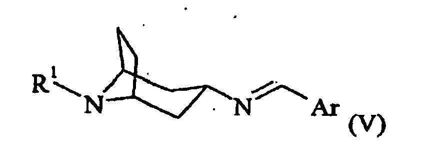 puis on hydrolyse et on convertit éventuellement en un sel d'addition d'acide.

2. Procédé selon la revendication 1 **caractérisé en ce que** R¹ représente un groupement choisi dans le groupe consistant en C₁-C₆-alkyle et phényl-C₁-C₃-alkyle.

3. Procédé selon la revendication 1 ou 2 **caractérisé en ce que** Ar représente un groupement phényle substitué une ou deux fois par C₁-C₆-alcoxy et/ou di-(C₁-C₆-alkyl)-amino.

4. Procédé selon l'une des revendications 1 à 3 **caractérisé en ce que** l'on conduit la réaction entre le composé de formule IIA et le composé de formule IIIA ou entre le composé de formule IIB et le composé de formule IIIB dans des conditions déshydratantes.

5. Procédé selon l'une des revendications 1 à 4 **caractérisé en ce que** l'on conduit l'hydrolyse du composé de formule V en présence d'un acide.

6. Procédé selon l'une des revendications 1 à 5 **caractérisé en ce que** les différentes étapes sont conduites chacune dans un diluant inerte choisi dans le groupe consistant en les hydrocarbures éventuellement halogénés, les amides, les nitriles, les sulfoxydes, les éthers et leurs mélanges.

7. 3-β-(arylméthylidène)-aminonortropane N-substitué de formule V 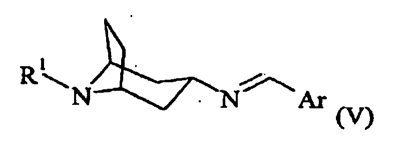 où
R¹ représente un groupement éventuellement substitué choisi dans le groupe consistant en C₁-C₈-alkyle, C₂-C₈-alcényle, C₃-C₈-cycloalkyle et C₆-C₁₀-aryl-C₁-C₈-alkyle ; et
Ar représente un groupement phényle éventuellement substitué ou un groupement hétéroaromatique à 5 ou 6 chaînons éventuellement substitué ayant au moins un hétéroatome choisi dans le groupe N, O et S.

8. 3-β-(arylméthylidène)-aminonortropane de formule V selon la revendication 7 où
R¹ représente un groupement choisi dans le groupe consistant en C₁-C₆-alkyle et phényl-C₁-C₃-alkyle, et
Ar représente phényle non substitué ou un groupement phényle substitué une ou deux fois par C₁-C₆-alcoxy et/ou di-(C₁-C₆-alkyl)-amino.
